Europäisches Patentamt

European Patent Office

Office européen des brevets

(19)

(11) Numéro de publication: **0 112 775**
**A1**

(12) **DEMANDE DE BREVET EUROPEEN**

(21) Numéro de dépôt: **83402474.7**

(22) Date de dépôt: **20.12.83**

(51) Int. Cl.³: **A 63 F 7/04, A 61 B 5/16**

(30) Priorité: **23.12.82 FR 8221633**

(43) Date de publication de la demande: **04.07.84**
**Bulletin 84/27**

(84) Etats contractants désignés: **AT BE CH DE GB IT LI LU NL SE**

(71) Demandeur: **Colart, André, Chemin des Fermes Villers sur Trie, F-60590 Serifontaine (FR)**

(72) Inventeur: **Colart, André, Chemin des Fermes Villers sur Trie, F-60590 Serifontaine (FR)**

(54) **Jouet-test de psychomotricité.**

(57) Jouet pouvant servir de test pour une évaluation d'une certaine psychomotricité. La transparence du jouet permet de suivre des yeux une bille percourant une piste transparente enroulée sur elle-même (fig. I) relié par un conduit qui donne la possibilité de sortir la bille par un trou (3) fait dans une face latérale d'une boite transparente dans laquelle est enfermée le jouet-test. Cette boite est une partie de la piste (7). Piste qui en son centre longitudinal est marqué d'une ligne continue.

EP 0 112 775 A1

## JOUET-TEST DE PSYCHOMOTRICITE

L'invention se rapporte à un jouet qui permet d'évaluer un certain degré de psychomotricité. Ce jeu-test est transparent.C'est sa transparence qui permet de suivre des yeux,la course d'une bille sur une piste enroulée sur elle-même.(Fig I)

Si la bille quitte la piste,elle tombe dans une boîte transparente qui enferme la dite piste.

La piste comporte des repères (4) qui permettent d'évaluer la distance parcourue par la bille.

Un trou (3) dans la boîte enfermant la piste, permet la sortie de la bille si celle-ci a parcouru la longueur de la piste jusqu'à son extrémité centrale (9). Dans le jouet présenté ici,la sortie ou l'entrée de la bille pour arriver sur la piste, se fait par le biais d'un petit conduit (2) reliant le trou (3) de la boîte à la piste.

L'endroit du départ de la bille est inscrit sur l'envers de la piste (6).La piste est également marquée par un trait continu déterminant son centre dans le sens de la longueur afin d'aider le joueur à ne pas quitter la piste.

Les repères de parcours précités sont ici constitués par des plis arrondis formant des angles droits. (4)

Ces plis permettent donc de compter chaque plan parcouru par la bille.

# 0112775

2

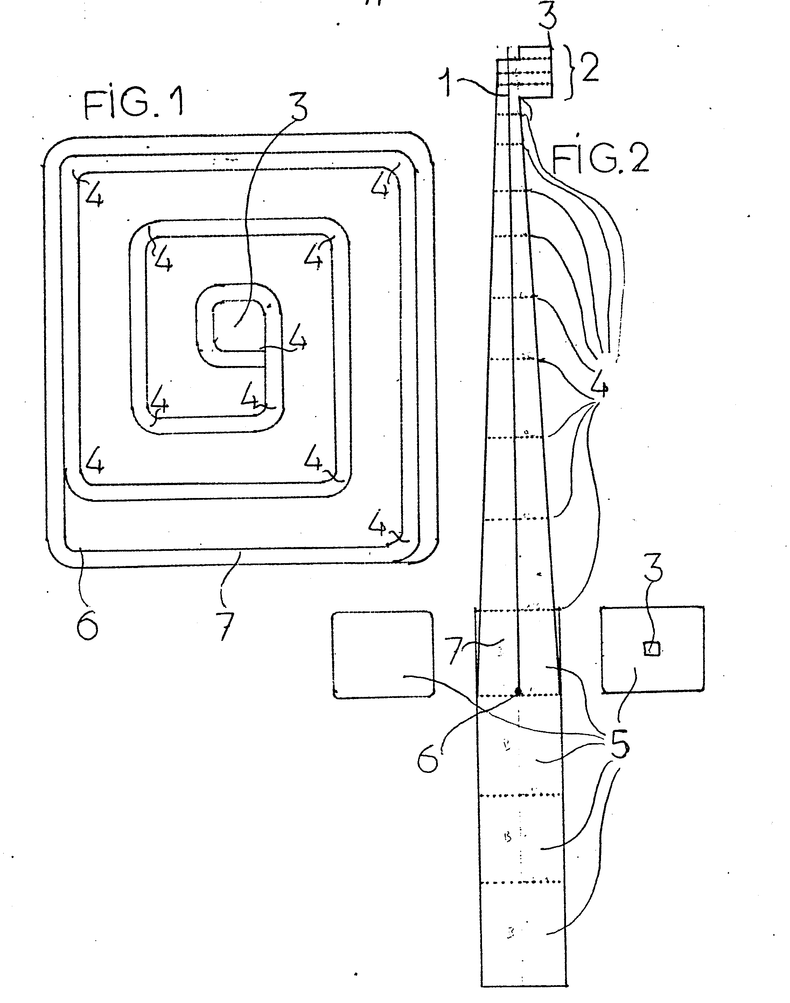

## REVENDICATIONS

I - Jouet pouvant servir de test pour une évaluation d'une certaine psychomotricité du type comportant une boite contenant une piste présentant un enroulement destiné à être parcouru par une bille d'un point de départ extérieur vers un trou central permettant de faire sortir la bille caractérisé en ce que d'une part,l'enroulement de la piste est réalisé sur elle-même de manière à ce que le déplacement de la bille s'effectue selon une ligne longitudinale obtenue par une piste constituée de plans disposés successivement à angle droit et reliés par des plis arrondis et en ce que d'autre part la dite piste est réalisée dans une matière transparente pour permettre de suivre malgré l'enroulement de la piste sur elle-même le déplacement de cette bille vers la zone centrale où la sortie de la dite bille s'effectue directement par un conduit aboutissant à la face latérale de la boite.

2 - Jouet-test selon la revendication I caractérisé en ce que cette piste se rétrécit progressivement vers la zone centrale.

3 - Jouet-test selon les revendications I et 2 caractérisé par une ligne continue disposée au centre longitudinal de la piste.

4 - Jouet-test selon les revendications I,2 et 3 caractérisé en ce que la piste est balisée par des repères de parcours au niveau des plis.

0112775

FIG.1

FIG.2

# RAPPORT DE RECHERCHE EUROPEENNE

0112775

Numéro de la demande

Office européen
des brevets

F P   83 40 2474

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| Y | US-A-3 702 191 (ZILIUS)<br>* figure 2; colonne 1,lignes 50-71; colonne 2, lignes 1-10 *<br><br>--- | 1-4 | A 63 F    7/04<br>A 61 B    5/16 |
| Y | CH-A- 257 436 (BEBIE)<br>* figures 4,5; revendication; sous-revendication 1 *<br><br>----- | 1-4 | |

DOMAINES TECHNIQUES
RECHERCHES (Int. Cl. ³)

A 63 F
G 09 B
A 61 B

Le présent rapport de recherche a été établi pour toutes les revendications

| Lieu de la recherche<br>LA HAYE | Date d'achèvement de la recherche<br>26-03-1984 | Examinateur<br>LOWE D. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82